# EUROPÄISCHE PATENTSCHRIFT

(11) **EP 1 235 821 B1**
(45) Veröffentlichungstag und Bekanntmachung des Hinweises auf die Patenterteilung: **21.04.2004**
(21) Anmeldenummer: 00990652.0
(22) Anmeldetag: 06.12.2000
(51) Int. Cl.: C07D 309/38, C07C 45/46, C07C 49/80

(54) **PYRONDERIVATE UND VERFAHREN ZU DEREN HERSTELLUNG**
PYRONE DERIVATIVES AND A METHOD FOR PRODUCING SAME
DERIVES DE PYRONE ET LEUR PROCEDE DE PRODUCTION

(30) Priorität: 06.12.1999 EP 99124350; 28.02.2000 US 185368 P
(43) Veröffentlichungstag der Anmeldung: 04.09.2002
(73) Patentinhaber: Lonza AG, 4052 Basel (CH)
(72) Erfinder: BRIEDEN, Walter, CH-3902 Brig-Glis (CH); GOTTSPONER, Michael, CH-3932 Visperterminen (CH)
(86) Internationale Anmeldenummer: PCT/EP2000/012243
(87) Internationale Veröffentlichungsnummer: WO 2001/040212

(56) Entgegenhaltungen:
- DE-A- 2 451 006
- FR-A- 2 238 696
- WERNER LÖWE: "SYNTHESIS AND CHARACTERISATION OF NEW CROWN TYPE COMPOUNDS WITH 4-PYRONE UNITS." JOURNAL OF HETEROCYCLIC CHEMISTRY., Bd. 34, Nr. 4, Juli 1997 (1997-07), Seiten 1173-8, XP002169941 HETEROCORPORATION. PROVO., US ISSN: 0022-152X in der Anmeldung erwähnt
- KAWADA A ET AL: "SCANDIUM TRIFLUORMETHANESULFONATE A NOVEL CATALYST FOR FRIEDEL- CRAFTS ACYLATION" SYNLETT, THIEME VERLAG, STUTTGART, DE, 1. Juli 1994 (1994-07-01), Seiten 545-546, XP002034429 ISSN: 0936-5214

## Beschreibung

Die vorliegende Erfindung betrifft ein Verfahren zur Herstellung von Pyronderivaten der allgemeinen Formel 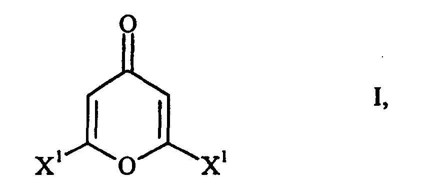 worin X¹ Halogenmethyl bedeutet, sowie ein neues Pyronderivat.

Pyronderivate der allgemeinen Formel I wie beispielsweise 2,6-Bis(chlormethyl)-4-pyron können wichtige Zwischenprodukte zur Herstellung von Kronenethern sein (W. Löwe et al., *J. Heterocyclic Chem*., 34, 1173 (1997).

Bisher sind nur das 2,6-Bis(brommethyl)-4-pyron und das 2,6-Bis(dibrommethyl)-4-pyron und ein Verfahren zu deren Herstellung bekannt (Löwe et al., ibid.). Dabei wird 2,6-Dimethyl-4-pyron mit *N*-Bromsuccinimid/Dibenzoylperoxid in Tetrachlormethan bromiert. Nachteilig bei diesem Verfahren ist, dass ein Gemisch verschiedener Produkte erhalten wird, aus welchem die genannten Verbindungen nur in sehr geringer Ausbeute isoliert werden können.

Aufgabe der vorliegenden Erfindung war daher, Verbindungen und ein Verfahren zu deren Herstellung bereit zu stellen, die zur Synthese von Kronenethern geeignet sind.

Erfindungsgemäss wird diese Aufgabe durch das Herstellungsverfahren gemäss Anspruch 1 und das neue Pyronderivat gemäss Anspruch 6 gelöst.

Das erfindungsgemässe Verfahren zur Herstellung der Pyronderivate der allgemeinen Formel 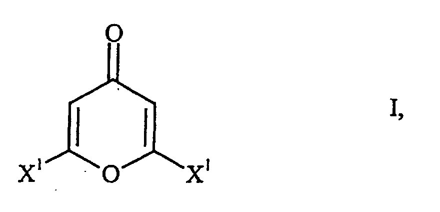 worin X¹ Halogenmethyl bedeutet, wird derart durchgeführt, dass man ein Acetessigsäurederivat der allgemeinen Formel 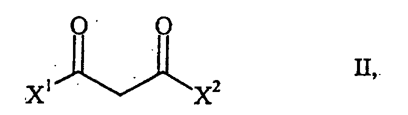 worin X¹ die genannte Bedeutung hat und X² Chlor oder Brom bedeutet, zunächst mit einer Lewis-Säure behandelt und dann mit Wasser in das gewünschte Produkt der allgemeinen Formel I umsetzt.

Unter Halogenmethyl wird hier und im folgenden Trihalogenmethyl, Dihalogenmethyl oder Monohalogenmethyl verstanden. Trihalogenmethyl und Dihalogenmethyl kann hierbei sowohl gleiche als auch verschiedene Halogenatome enthalten. Beispiele für Trihalogenmethyl sind Trifluormethyl und Tribrommethyl. Beispiele für Dihalogenmethyl sind Dichlormethyl, Dibrommethyl, Difluormethyl und Beispiele für Monohalogenmethyl sind Fluormethyl, Chlormethyl, Brommethyl oder Iodmethyl. Als Halogenmethyl sind beispielsweise auch Difluormonochlormethyl, Dibrommonochlormethyl und Dichlormonofluormethyl geeignet.

Als Lewis-Säuren sind Scandiumsalze wie z. B. Scandiumtrifluormethansulfonat oder Scandiumsulfat, Lanthansalze wie z. B. Lanthantrifluormethansulfonat, Yttriumsalze oder BF₃·O(C₂H₅)₂ geeignet. Vorzugsweise wird als Lewis-Säure Scandiumtrifluormethansulfonat eingesetzt.

Vorteilhaft wird die Lewis-Säure in einer Menge von 0,01 bis 3 mol, vorzugsweise in einer Menge von 0,05 bis 0,5 mol, pro mol Acetessigsäurederivat der allgemeinen Formel II eingesetzt.

Vorzugsweise wird die Umsetzung in einem halogenierten organischen Lösungsmittel durchgeführt. Als halogenierte organische Lösungsmittel können Halogenkohlenwasserstoffe wie Dichlormethan, Chloroform, Tetrachlormethan, 1,1,1-Trichlorethan oder 1,2-Dichlorethan eingesetzt werden. Besonders bevorzugt ist Dichlormethan.

Zweckmässig wird die Umsetzung bei einer Temperatur von -20 bis 60 °C, vorzugsweise bei einer Temperatur von 0 bis 25 °C, durchgeführt.

Die Umsetzung in der ersten und zweiten Stufe wird zweckmässig unter Inertgasatmosphäre wie beispielsweise unter Stickstoffatmosphäre durchgeführt.

Nach einer Umsetzungszeit von üblicherweise 1 bis 8 Tagen können die Pyronderivate der allgemeinen Formel I mittels üblicher Aufarbeitungsmethoden isoliert werden.

Das Pyronderivat der allgemeinen Formel I 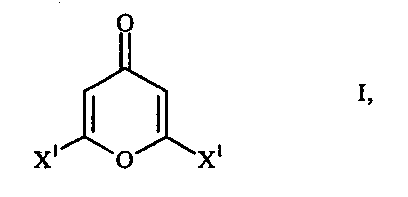 worin X¹ Chlormethyl bedeutet, ist eine neue Verbindung und ebenfalls Gegenstand der Erfindung.

Das folgende Beispiel verdeutlicht die Durchführung des erfindungsgemässen Verfahrens und die Herstellung der erfindungsgemässsen Verbindung.

### Beispiel

### Herstellung von 2,6-Bis(chlormethyl)-4-pyron

4-Chloracetoacetylchlorid (14,8 g, 33,4 mol) in Dichlormethan (35 %ige Lösung) und Scandiumtrifluormethansulfonat (1,45 g, 2,5 mol) wurden bei 25 °C 6,5 Tage unter Stickstoffatmosphäre gerührt. Dann wurde Wasser (10 g, 0,55 mol) hinzugegeben und die organische Phase wurde eingeengt. Man erhielt 4,2 g Produkt (nach GC 68%iges Rohprodukt). Nach Chromatographie über eine Kieselgelsäule wurden 0,7 g reines Produkt entsprechend einer Ausbeute von 10,8 % erhalten.

## Patentansprüche

1. Verfahren zur Herstellung von Pyronderivaten der allgemeinen Formel 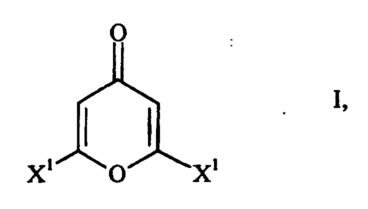 worin X¹ Halogenmethyl bedeutet, **dadurch gekennzeichnet, dass** man ein Acetessigsäurederivat der allgemeinen Formel 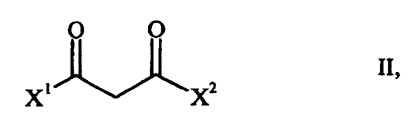 worin X¹ die genannte Bedeutung hat und X² Chlor oder Brom bedeutet, zunächst mit einer Lewis-Säure behandelt und dann mit Wasser in das gewünschte Produkt (I) überführt.

2. Verfahren nach Anspruch 1, **dadurch gekennzeichnet, dass** man als Lewis-Säure Scandiumtrifluormethansulfonat einsetzt.

3. Verfahren nach Anspruch 1 oder 2, **dadurch gekennzeichnet, dass** man die Lewis-Säure in einer Menge von 0,01 bis 3 mol pro mol Acetessigsäurederivat (II) einsetzt.

4. Verfahren nach mindestens einem der Ansprüche 1 bis 3, **dadurch gekennzeichnet, dass** die Umsetzung in der ersten Stufe in einem halogenierten organischen Lösungsmittel durchgeführt wird.

5. Verfahren nach mindestens einem der Ansprüche 1 bis 4, **dadurch gekennzeichnet, dass** die Umsetzung bei einer Temperatur von -20 bis +60 °C durchgeführt wird.

6. Pyronderivat der Formel 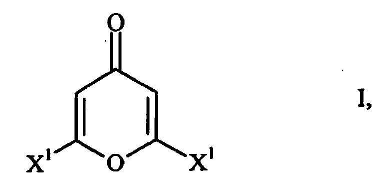 worin X¹ Monochlormethyl bedeutet.

## Claims

1. Process for the preparation of pyrone derivatives of the general formula 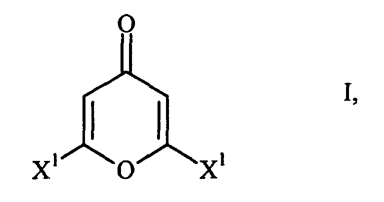 in which X¹ is halomethyl, **characterized in that** an acetoacetic acid derivative of the general formula 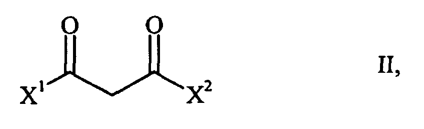 in which X¹ has the meaning mentioned and X² is chlorine or bromine, is first treated with a Lewis acid and then converted into the desired product I using water.

2. Process according to Claim 1, **characterized in that** the Lewis acid employed is scandium trifluoromethanesulfonate.

3. Process according to Claim 1 or 2, **characterized in that** the Lewis acid is employed in an amount from 0.01 to 3 mol per mole of acetoacetic derivative (II).

4. Process according to at least one of Claims 1 to 3, **characterized in that** the reaction in the first stage is carried out in a halogenated organic solvent.

5. Process according to at least one of Claims 1 to 4, **characterized in that** the reaction is carried out at a temperature of -20 to +60°C.

6. Pyrone derivative of the formula 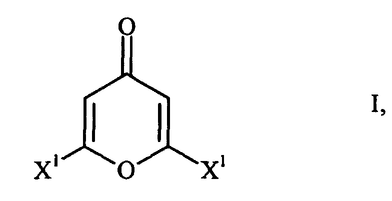 in which X¹ is monochloromethyl.

## Revendications

1. Procédé pour la préparation de dérivés de pyrone de formule générale 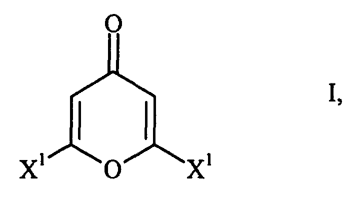 dans laquelle X¹ représente un groupe halogénométhyle, **caractérisé en ce que** d'abord on traite par un acide de Lewis un dérivé d'acide acétoacétique de formule générale 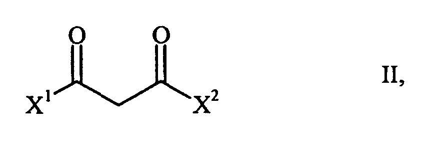 dans laquelle X¹ a la signification indiquée et X² représente le chlore ou le brome, et on le convertit ensuite, avec de l'eau, en le produit (I) recherché.

2. Procédé selon la revendication 1, **caractérisé en ce qu'**on utilise en tant qu'acide de Lewis du trifluorométhanesulfonate de scandium.

3. Procédé selon la revendication 1 ou 2, **caractérisé en ce qu'**on utilise l'acide de Lewis en une quantité de 0,01 à 3 moles par mole de dérivé d'acide acétoacétique (II).

4. Procédé selon au moins l'une des revendications 1 à 3, **caractérisé en ce que** dans la première étape on effectue la réaction dans un solvant organique halogéné.

5. Procédé selon au moins l'une des revendications 1 à 4, **caractérisé en ce qu'**on effectue la réaction à une température de -20 à +60°C.

6. Dérivé de pyrone de formule 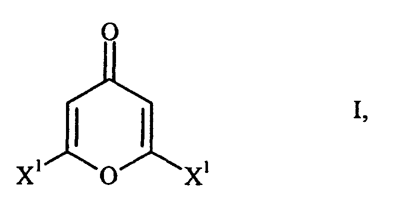 dans laquelle X¹ représente le groupe monochlorométhyle.
